# EUROPEAN PATENT SPECIFICATION

(11) **EP 1 940 315 B1**
(45) Date of publication and mention of the grant of the patent: **11.05.2011**
(21) Application number: 06740310.5
(22) Date of filing: 31.03.2006
(51) Int. Cl.: A61F 2/90

(54) **STENT WITH EXPANDING SIDE BRANCH GEOMETRY**
STENT MIT EXPANDIERENDER SEITENAST-GEOMETRIE
STENT A GEOMETRIE DE BRANCHE LATERALE EXTENSIBLE

(30) Priority: 29.08.2005 US 214529
(43) Date of publication of application: 09.07.2008
(73) Proprietor: Boston Scientific Limited, Hastings Christ Church (BB)
(72) Inventor: GREGORICH, Daniel, St. Louis Park, Minnesota 55416 (US); GROTHEIM, Kevin, 55114 St. Paul, MN (US); MILLER, Matthew J., Stillwater, Minnesota 55082 (US)
(74) Representative: Hauck Patent- und Rechtsanwälte
(86) International application number: PCT/US2006/012147
(87) International publication number: WO 2007/027201

(56) References cited:
- WO-A-01/97717
- US-A- 6 059 824
- US-A1- 2002 193 873
- US-A1- 2004 088 007

## Description

### BACKGROUND OF THE INVENTION

### Field of the Invention

In some embodiments this invention relates to implantable medical devices. Some embodiments are directed to delivery systems, such as catheter systems of all types, which are utilized in the delivery of such devices.

### Description of the Related Art

A stent is a medical device introduced to a body lumen and is well known in the art. Typically, a stent is implanted in a blood vessel at the site of a stenosis or aneurysm endoluminally, i.e. by so-called "minimally invasive techniques" in which the stent in a radially reduced configuration, optionally restrained in a radially compressed configuration by a sheath and/or catheter, is delivered by a stent delivery system or "introducer" to the site where it is required. The introducer may enter the body from an access location outside the body, such as through the patient's skin, or by a "cut down" technique in which the entry blood vessel is exposed by minor surgical means.

Stents, grafts, stent-grafts, vena cava filters, expandable frameworks, and similar implantable medical devices, collectively referred to hereinafter as stents, are radially expandable endoprostheses which are typically intravascular implants capable of being implanted transluminally and enlarged radially after being introduced percutaneously. Stents may be implanted in a variety of body lumens or vessels such as within the vascular system, urinary tracts, bile ducts, fallopian tubes, coronary vessels, secondary vessels, etc. Stents may be used to reinforce body vessels and to prevent restenosis following angioplasty in the vascular system. They may be self-expanding, expanded by an internal radial force, such as when mounted on a balloon, or a combination of self-expanding and balloon expandable (hybrid expandable).

Stents may be created by methods including cutting or etching a design from a tubular stock, from a flat sheet which is cut or etched and which is subsequently rolled or from one or more interwoven wires or braids.

Within the vasculature, it is not uncommon for stenoses to form at a vessel bifurcation. A bifurcation is an area of the vasculature or other portion of the body where a first (or parent) vessel is bifurcated into two or more branch vessels. Where a stenotic lesion or lesions form at such a bifurcation, the lesion(s) can affect only one of the vessels (i.e., either of the branch vessels or the parent vessel) two of the vessels, or all three vessels. Many prior art stents however are not wholly satisfactory for use where the site of desired application of the stent is juxtaposed or extends across a bifurcation in an artery or vein such, for example, as the bifurcation in the mammalian aortic artery into the common iliac arteries.

US 6,059,824A discloses a main stent having a lateral opening with detents or inlets spaced about the annular extent of the opening. Said detents or inlets mate with a collateral stent having the other of detents or inlets at one of its ends.

The art referred to and/or described above is not intended to constitute an admission that any patent, publication or other information referred to herein is "prior art" with respect to this invention, hi addition, this section should not be construed to mean that a search has been made or that no other pertinent information as defined in 37 C.F.R. [section]1.56(a) exists.

Without limiting the scope of the invention as claimed a brief summary of some of the claimed embodiments of the invention is set forth below. Additional details of the summarized embodiments of the invention and/or additional embodiments of the invention may be found in the Detailed Description of the invention below.

A brief abstract of the technical disclosure in the specification is provided as well only for the purposes of complying with 37 C.F.R. 1.72. The abstract is not intended to be used for interpreting the scope of the claims.

### BRIEF SUMMARY OF THE INVENTION

This invention contemplates a number of embodiments where any one, any combination of some, or all of the embodiments can be incorporated into a stent and/or a stent delivery system and/or a method of use.

At least one embodiment of the invention is directed to a stent having a generally tubular first stent body positioned within a circumferential plane defining a first lumen having deployed and undeployed states, expanded and unexpanded states, and an expanding side branch assembly also having a deployed and undeployed state where the side branch assembly in the unexpanded state is completely or substantially along or within the first stent body and in the expanded state the side branch assembly defines a second lumen partially within the first lumen and partially without.

At least one embodiment of the invention is directed to a stent having a second fluid lumen which is restrained from expansion by a member of the first stent body in the unexpanded state and in the expanded state is not restrained from expansion by the member of the first stent body.

In at least one possible embodiment the side branch assembly is self-expanding.

In at least one possible embodiment the side branch assembly is balloon expandable.

In at least one possible embodiment, the undeployed side branch is positioned entirely within the lumen of the first stent body.

In at least one embodiment, the self expansion mechanism includes biased members of the side branch assembly which in the unexpanded state are restrained by blocking struts of the main stent body and in the expanded state are released when restraining struts of the main stent body are withdrawn.

In at least one embodiment, the second stent body is comprised of a plurality of side branch projections and a mounting ring, each of the side branch projections having a free end and an engaged end with the engaged end is engaged to the mounting ring.

In at least one embodiment, the stent includes a mounting ring engaged to the first stent body by at least one engagement region adjacent to the first side branch opening.

In at least one embodiment, the second fluid lumen is a second stent body.

In at least one embodiment, the second stent body also comprises a plurality of engagement members, the engagement members extending from a mounting ring to at least one engagement region of the first stent body.

In at least one embodiment, the first stent body is comprised of a plurality of interconnected first stent members, adjacent first stent members defining a plurality of openings through the first stent body in fluid communication with the lumen, at least one of the openings being the first side branch opening.

In at least one embodiment the stent has a plurality of side branch openings each with an area.

In at least one embodiment the stent has a plurality of side branch openings each with an area and the area of at least one side branch opening is greater than or smaller than that of each of the remaining openings.

In at least one embodiment the stent has a plurality of side branch openings and the first side branch opening and the second side branch opening are coaxially positioned relative to one another.

In at least one embodiment, the side branch assembly is a second stent body which in the undeployed state comprises a substantially tubular shape and has a common longitudinal axis with first stent body.

In at least one embodiment, the first stent body has an end-to-end length and the second stent body has an end-to-end length, and the end-to-end length of the second body is shorter than the end-to-end length of the first stent body.

In at least one embodiment, when in the undeployed state, no first stent body members are positioned across the second side branch opening.

In at least one embodiment, the self expansion mechanism is a constrained by a sheath surrounding the stent until after expansion, which when withdrawn, allows the side branch assembly to self expand.

In at least one embodiment, the self expansion mechanism is a plurality of biased wires, restrained by the stent geometry in the unexpanded state but which are released when the stent expands either pulls or pushes the side branch assembly away from the main stent body.

In at least one embodiment, the self expansion mechanism is a plurality of hooks, connecting the side branch to the main stent body which are pushed by the stent expansion into rotating which pushes the side branch away from the main stent body.

These and other embodiments which characterize the invention are pointed out with particularity in the claims annexed hereto and forming a part hereof. However, for further understanding of the invention, its advantages and objectives obtained by its use, reference should be made to the drawings which form a further part hereof and the accompanying descriptive matter, in which there is illustrated and described a embodiments of the invention.

### BRIEF DESCRIPTION OF THE SEVERAL VIEWS OF THE DRAWING(S)

A detailed description of the invention is hereafter described with specific reference being made to the drawings.
FIG. 1 is a view of an unexpanded stent having a first stent body containing an undeployed side branch assembly completely within the first stent body.
FIG. 2 is a view of an expanded stent having a first stent body and an expanded second stent body partially within and partially without the first stent body.
FIG. 3 is a view of a stent in the expanded state where a self expanding side branch assembly is partially within a first fluid lumen and has formed a second fluid lumen.
FIG. 4 is a view of a side branch assembly in the unexpanded state where the structure is restrained by members of the main stent body.
FIG. 5 is a view of a side branch in the expanded state where the assembly has formed a second lumen, partially within the main body and partially without.
FIG. 6 is a view of a stent in the unexpanded state in which the stent has a side branch assembly that is completely within the first stent body.
FIG. 7 is a view of an expanded stent with a side branch assembly in the expanded state which forms a second fluid lumen partially within the first stent body and partially without.
FIG. 8 is a cut away view showing the second fluid lumen partially within the first stent body.
FIG. 9 is a view of stent with a side branch assembly with wires.
FIG. 10 is a view of stent with a side branch assembly with hook guides.

### DETAILED DESCRIPTION OF THE INVENTION

While this invention may be embodied in many different forms, there are described in detail herein specific embodiments of the invention. This description is an exemplification of the principles of the invention and is not intended to limit the invention to the particular embodiments illustrated.

For the purposes of this disclosure, like reference numerals in the figures shall refer to like features unless otherwise indicated.

Depicted in the figures are various aspects of the invention. Elements depicted in one figure may be combined with, and/or substituted for, elements depicted in another figure as desired.

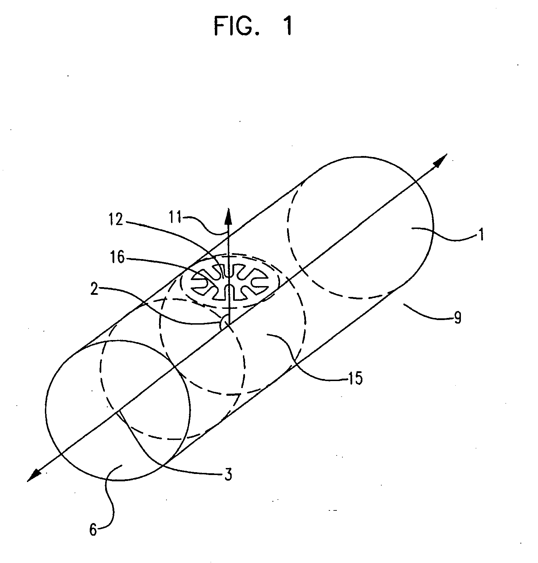

Referring now to FIG. 1 there is shown an unexpanded stent 9 extending along a first longitudinal axis 3 having a first stent body 1. The first stent body 1 can be constructed at least partially out of a number of materials including but not limited to polymers, stainless steel, platinum, gold, cobalt, chromium, niobium etc. It can also be constructed out of one or more combinations and/or alloys of these materials. The figure also shows that contained with the first stent body 1 there is a side branch assembly 15 adjacent to a side branch opening 12 within the unexpanded first stent body 1. This side branch assembly 15 is a second stent body within the first stent body. While unexpanded this side branch assembly is completely or substantially contained within the first stent body 1. The side branch assembly has a second longitudinal axis 11 which forms an oblique angle 2 with the first longitudinal axis 3. Although the figure shows a single side branch opening and a singe side branch assembly, there can be multiple side branch openings and side branch assemblies. The sizes of the side branches can vary as well having larger, smaller or the same area, end-on-end length, or circumference in the extended or unextended states. Multiple side branch openings can be positioned anywhere along the length of the first stent body 1 and can be coaxially positioned relative to one another.

In at least one embodiment, the second fluid lumen is a second stent body which in the undeployed state can also comprise a substantially tubular shape, the lumen and the longitudinal axis of the first stent body being common to the second stent body.

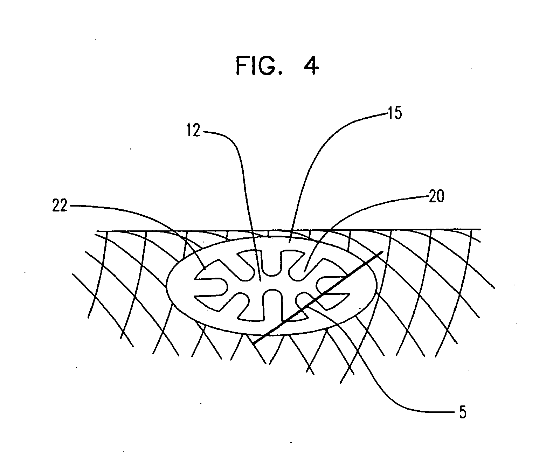

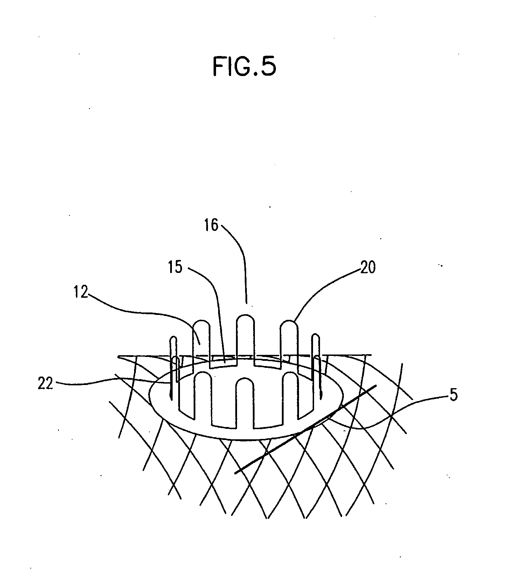

This illustration features one possible embodiment where the side branch opening 12 is covered. In FIG. 1 the covering is a plurality of projecting members 16 each with an engaged end attached to the first body of the stent 1 having a plurality of strut members 5 and an unattached free end, the members 16 covering the side branch opening 12 through which the second fluid lumen 20 will project. When the first stent body is expanded, the projecting members 16 can change their angular orientation to form at least part of a second fluid lumen 20 in fluid communication with a fist fluid lumen 6 of the first stent body 1. In some embodiments, such as are shown in FIGs. 4 and 5, struts 5 or other components of the main stent body 1 can also be configured to cover and/or retain projecting members 16 in the unexpanded state and to pull away from the projecting members 16 in the expanded state.

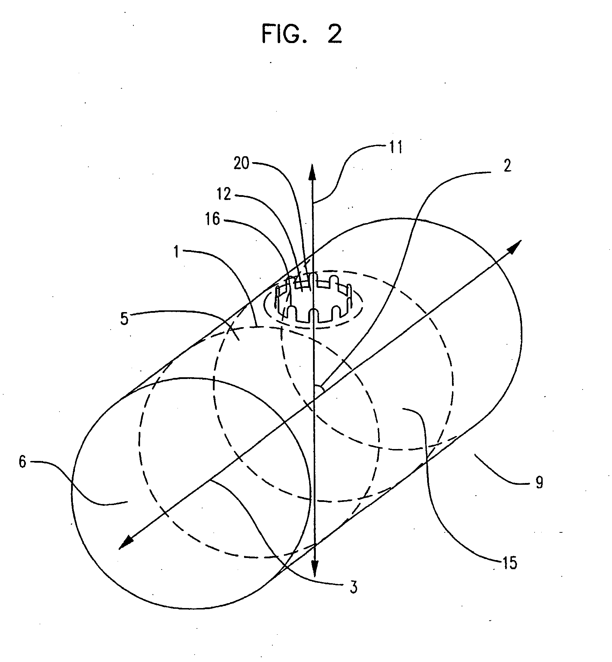

The effects of expanding the stent 9 is illustrated in FIG. 2 where there is shown a stent 9 in an expanded state. The side branch assembly 15 is partially extended out of the first stent body 1 forming a side branch projection and is partially within the first fluid lumen 6 of the first stent body 1. This side branch assembly 15 defines a second fluid lumen 20 in fluid communication with the first fluid lumen 6. In this illustration there is shown the possible embodiment the projection members 16 of the side branch assembly 15 have been angled away from the main stent body 1 and comprise the portion of the second fluid lumen 20 that extends outside of the first stent body 1. The second fluid lumen 20 can be formed with or without projecting members 16. In other embodiments the projecting members 16 could have defined at least a portion of the second fluid lumen or they could have been pulled aside from the extension of the side branch assembly 15. The side branch assembly 15 can increase metal coverage in the ostium of the side vessel branch and can restrain the projecting members 16 from projecting in the unexpanded state.

The expansion of either or both of the main stent body 1 or the side branch assembly 15 could have been accomplished by balloon expansion or self expansion. The side branch assembly can be constructed out of any one or any combination of materials including but not limited to polymers, stainless steel, nickel, titanium, niobium etc. The side branch projection extends along a second longitudinal axis 1 forming an oblique angle 2 to the first longitudinal axis. For the purposes of this application, the term "oblique" refers to an angle of between 1 and 180 degrees and explicitly includes angles of about 90 degrees.

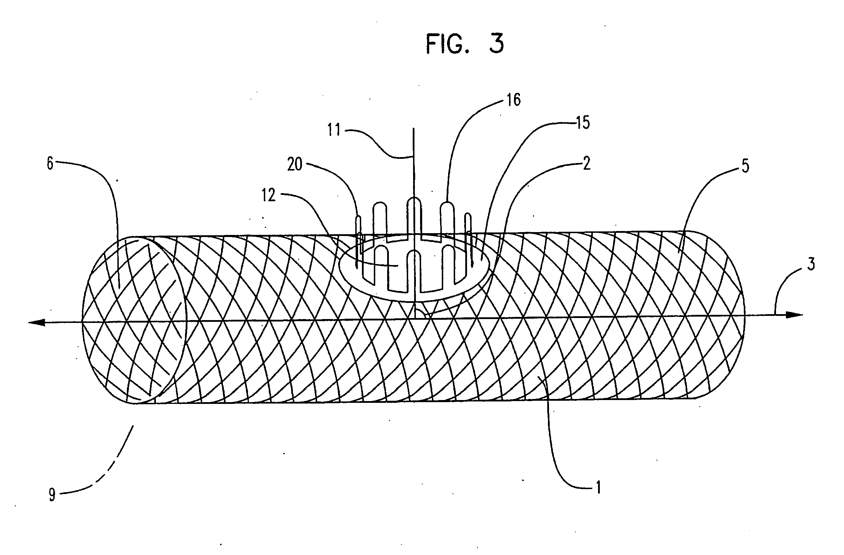

Referring now to FIG. 3 there is shown an expanded stent 9 having a plurality of strut members 5 with a side branch assembly 15 where a portion of the second fluid lumen 20 is formed by a plurality of projection members 16 angled away from the longitudinal axis 3 of the first stent body 1. The projecting members 16 form a second fluid lumen 6 generally extending along a second longitudinal axis 11 forming an angle 2 to the first longitudinal axis 3. In some embodiments, the first stent body 1 and the side branch assembly 15 could be constructed out of struts, flexible members, flaps, plates, and any number of other known structures and are contemplated by this application.

In at least one embodiment, an example of which is shown in FIG .4, the side branch assembly comprises a mounting ring 22. This mounting ring 22 encircles the side branch opening 12 and is connected to the side branch projection of the second fluid lumen 20. A side branch projection is made up of a plurality of projecting members 16, the mounting ring 22 is connected to the engaged end of a projecting member 16 and the other end of a projecting member is a free end. The mounting ring 22 is not limited to a circular or even elliptical shape. The mounting ring 22 may be of any size and/or shape sufficient to support the side branch projection and retain the assembly 15 to the first stent body 1, such as in the manner shown in FIGs. 4 and 5.

The mounting ring 22 is connected to the main stent body 1 at an engagement region adjacent to the side branch opening 12. The mounting ring 22 has also a plurality of engagement members extending from the mounting ring 22 to at least one engagement region of the first stent body I.

The stent 9 can be constructed to have two or more side branch assembles 15 and side branch openings 12. In addition, the multiple side branch openings can have differing areas and the different side branch projections can have differing lengths. The multiple side branches can be coaxially located or positioned anywhere along the surface of the first stent body 1.

The side branch assembly 15 can be constructed in such a manner as to be self expanding. In the unexpanded state, this biased side branch assembly 15 is restrained, but once the restraint is removed, the side branch assembly 15 self expands and forms a second fluid lumen 16.

In at least one embodiment, a sheath encapsulates the stent 9 in the unexpanded state and can act to restrain a biased self expanding side branch 15. In this possible embodiment, once the sheath is removed, the now unrestrained side branch assembly 15 can self expand. This mechanism allows for the side branch to expand before, after, or at substantially the same time that the main stent body 1 expands.

In at least one embodiment, in the unexpanded state, at least one strut member 5 of the main stent body 1 is positioned in such a manner as to block self expansion of the side branch assembly 15. As the stent expands, the strut member 5 is pushed away from the side branch assembly 15, which allows it to self-expand.

Referring now to FIG. 4 there is shown an embodiment of an unexpanded side branch 15 on a mounting ring 22 in which at least one projecting member 16 is restrained by at least one strut 5 of the first stent body 1, such as is shown in FIG. 3. As the stent 9 expands, the strut(s) 5 will tend to move off of the projecting member 20 of the side branch assembly 15 to allow the projecting member or members 16 to expand, such as in the manner depicted in FIG. 5.

In embodiments wherein the side branch assembly 15 is configured to be self expanding, this movement will release the projecting member 16 to self-expand. In the case of a balloon expandable side branch assembly 15, movement of the strut(s) 5 away would allow balloon expansion to extend the side branch assembly 15. As mentioned before, this inventive concept is not limited to stents comprising struts and can be accomplished with any stent structure in which in the unexpanded state the stent has a structural component adjacent to the structural components of the side branch assembly and in the expanded state the structural components are moved away from the side branch assembly.

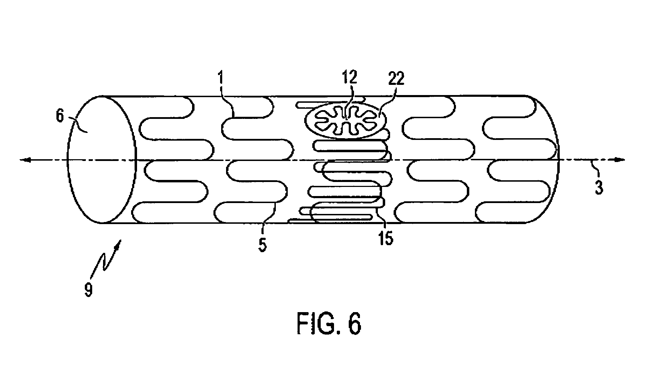

Referring now to FIG. 6 there is shown an unexpanded stent 9 comprising a plurality of interconnected struts 5 with a mounting ring 22 engaged to the side branch assembly 15. This mounting ring 22 can be biased to push the side branch assembly 15 away from the main body 1 of the stent 9. When the stent 9 is unexpanded, the mounting ring 22 can be crimped below the strut members 5 of the main stent body. The mounting ring 22 can be positioned anywhere on the stent main body 1 in structural communication with the side branch assembly 15. The mounting ring 22 can also comprise a column of strut members integrated into the structure of the main stent body 1. The mounting ring 22 provides reinforced support which can assist the side branch assembly 15 increase metal coverage in the area above and the area below the vessel ostium, and can assist the side branch assembly 15 restrain the projecting members 16 from projecting in the unexpanded state.

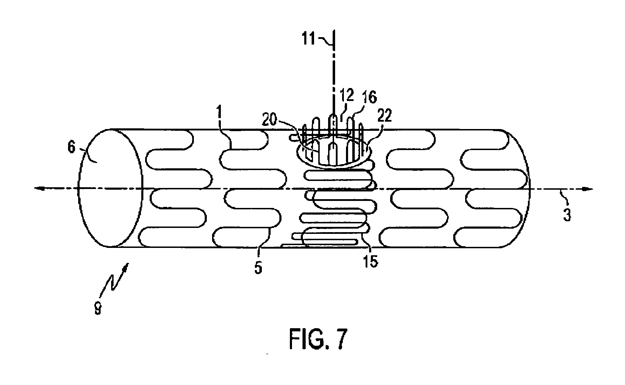

FIG. 7 illustrates the stent of FIG. 6 in the expanded state showing that the mounting ring 30 when no longer being restrained by the struts of the main stent body 1 connects extended side branch assembly 15 to the main stent body 1 allowing the formation of a second fluid lumen 20.

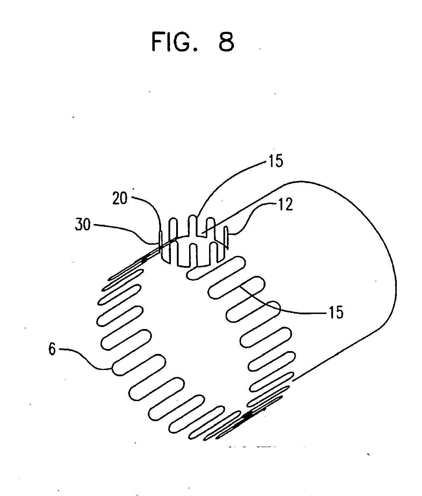

Now referring to FIG. 8 there is shown a view of the side branch assembly 15 viewed from within the main stent body 1. This illustration shows how the second fluid lumen 20 lies partially within the first fluid lumen 6 and also comprises a projecting branch defining a portion of the second fluid lumen 20 which is external to the expanded first stent body 1. The two fluid lumens (6 and 20) are in fluid communication with each other.

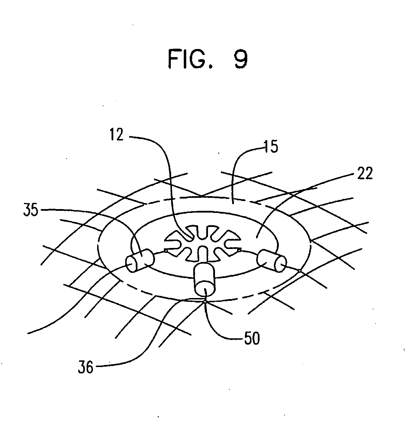

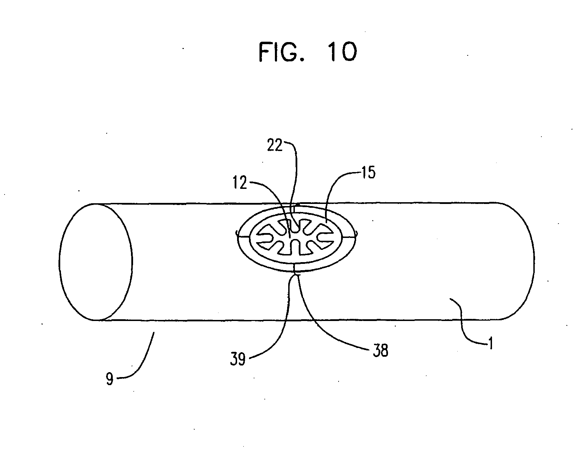

The mounting ring 22 of the side branch assembly is also connected to the first stent body 1 by a plurality of engagement members. Embodiments of this are illustrated in FIGs 9 and 10.

In FIG. 9 there is shown an illustration of a stent 9 having a side branch assembly 15 comprising at least one wire 36 connected to the main stent body 1 by a at wire-stent connector 50. This wire-stent connector can connect the side branch assembly 15 to the first stent body 1. An optional wire guide 35 can be added to the stent but is not essential to this embodiment.

This wire-stent connector can also function as an additional or alternative mechanism for expanding the side branch projection by pushing the side branch projection away from the circumferential plane of the stent. As the main stent body expands, the expansion moves the wire-stent connector 50 which can either push or pull the wire 36 to move the side branch assembly 15 into an expanded state.

In FIG. 10 there is shown an illustration of a stent 9 having a side branch assembly 15 comprising at least one hook 38 connected to the main body of the stent 1 in the unexpanded state. As the stent expands, the relative location of the main body-hook connection 39 to the side branch 15 changes which exerts a torsional force on the hook 38. The hook 38 responds to this torsional force by rotating in a direction that pushes side branch 15 away from the main stent body 1.

The inventive stents may be made from any suitable biocompatible materials including one or more polymers, one or more metals or combinations of polymer(s) and metal(s). Examples of suitable materials include biodegradable materials that are also biocompatible. By biodegradable is meant that a material will undergo breakdown or decomposition into harmless compounds as part of a normal biological process. Suitable biodegradable materials include polylactic acid, polyglycolic acid (PGA), collagen or other connective proteins or natural materials, polycaprolactone, hylauric acid, adhesive proteins, co-polymers of these materials as well as composites and combinations thereof and combinations of other biodegradable polymers. Other polymers that may be used include polyester and polycarbonate copolymers. Examples of suitable metals include, but are not limited to, stainless steel, titanium, tantalum, platinum, tungsten, gold and alloys of any of the above-mentioned metals. Examples of suitable alloys include platinum-iridium alloys, niobium alloys, cobalt-chromium alloys including Elgiloy and Phynox, MP35N alloy and nickel-titanium alloys, for example, Nitinol.

The inventive stents may be made of shape memory materials such as superelastic Nitinol or spring steel, or may be made of materials which are plastically deformable. In the case of shape memory materials, the stent may be provided with a memorized shape and then deformed to a reduced diameter shape. The stent may restore itself to its memorized shape upon or after being heated to a transition temperature and having any restraints removed therefrom.

The inventive stents may be created by methods including cutting or etching a design from a tubular stock, from a flat sheet which is cut or etched and which is subsequently rolled or from one or more interwoven wires or braids. Any other suitable technique which is known in the art or which is subsequently developed may also be used to manufacture the inventive stents disclosed herein.

In some embodiments the stent, the delivery system or other portion of the assembly may include one or more areas, bands, coatings, members, etc. that is (are) detectable by imaging modalities such as X-Ray, MRI, ultrasound, etc. In some embodiments at least a portion of the stent and/or adjacent assembly is at least partially radiopaque.

In some embodiments the at least a portion of the stent is configured to include one or more mechanisms for the delivery of a therapeutic agent. Often the agent will be in the form of a coating or other layer (or layers) of material placed on a surface region of the stent, which is adapted to be released at the site of the stent's implantation or areas adjacent thereto.

A therapeutic agent may be a drug or other pharmaceutical product such as non-genetic agents, genetic agents, cellular material, etc. Some examples of suitable non-genetic therapeutic agents include but are not limited to: anti-thrombogenic agents such as heparin, heparin derivatives, vascular cell growth promoters, growth factor inhibitors, Paclitaxel, etc. Where an agent includes a genetic therapeutic agent, such a genetic agent may include but is not limited to: DNA, RNA and their respective derivatives and/or components; hedgehog proteins, etc. Where a therapeutic agent includes cellular material, the cellular material may include but is not limited to: cells of human origin and/or non-human origin as well as their respective components and/or derivatives thereof. Where the therapeutic agent includes a polymer agent, the polymer agent may be a polystyrene-polyisobutylene-polystyrene triblock copolymer (SIBS), polyethylene oxide, silicone rubber and/or any other suitable substrate.

The above disclosure is intended to be illustrative and not exhaustive. This description will suggest many variations and alternatives to one of ordinary skill in this art. The various elements shown in the individual figures and described above may be combined or modified for combination as desired. All these alternatives and variations are intended to be included within the scope of the claims where the term "comprising" means "including, but not limited to".

Further, the particular features presented in the dependent claims can be combined with each other in other manners within the scope of the invention such that the invention should be recognized as also specifically directed to other embodiments having any other possible combination of the features of the dependent claims. For instance, for purposes of claim publication, any dependent claim which follows should be taken as alternatively written in a multiple dependent form from all prior claims which possess all antecedents referenced in such dependent claim if such multiple dependent format is an accepted format within the jurisdiction (e.g. each claim depending directly from claim 1 should be alternatively taken as depending from all previous claims). In jurisdictions where multiple dependent claim formats are restricted, the following dependent claims should each be also taken as alternatively written in each singly dependent claim format which creates a dependency from a prior antecedent-possessing claim other than the specific claim listed in such dependent claim below.

This completes the description of the invention. Those skilled in the art may recognize other equivalents to the specific embodiment described herein which equivalents are intended to be encompassed by the claims attached hereto.

## Claims

1. A stent assembly having an undeployed state and a deployed state, the assembly comprising:
• a substantially tubular, first stent body (1) defining a lumen (6) positioned within a circumferential plane and having a longitudinal axis (3) therethrough; and
• a second stent body, in the undeployed state the second stent body at least partially positioned within the lumen (6) of the first stent body (1), the first stent body (1) defining a first side branch opening (12) in fluid communication with the lumen (6), the second stent body comprising a plurality of side branch projecting members (16), the projecting members (16) defining a second side branch opening (12) in fluid communication with the first side branch opening and the lumen, in the deployed state the projecting members (16) extend through the first side branch opening (12) and form an oblique angle relative to the longitudinal axis (3), wherein the second stent body is comprised of the plurality of side branch projecting members (16) and a mounting ring (22), each of the side branch projecting members (16) having a free end and an engaged end, wherein the engaged end is engaged to the mounting ring (22) **characterised in that** in the mounting ring (22) is engaged the first stent body (1) by at least one engagement region adjacent to the first side branch opening (12), and the second stent body is further comprised of a plurality of engagement members, the engagement members extending from the mounting ring (22) to the at least one engagement region of the first stent body (1).

2. The stent assembly of claim 1 wherein the first stent body (1) is a balloon expandable stent.

3. The stent assembly of claim 2 wherein the second stent body is a self-expandable stent.

4. The stent assembly of claim 3 wherein the first stent body (1) is at least partially constructed of at least one material of the group consisting of: stainless steel, platinum, gold, cobalt, chromium, niobium, titanium, and any combinations or alloys thereof.

5. The stent assembly of claim 3 wherein the plurality of side branch projecting members (16) are at least partially constructed from at least one material of the group consisting of: nickel, titanium, niobium and any combinations or alloys thereof.

6. The stent assembly of claim 1 wherein the first stent body (1) is comprised of a plurality of interconnected first stent members, adjacent first stent members defining a plurality of openings through the first stent body in fluid communication with the lumen (6), at least one of the openings being the first side branch opening (12).

7. The stent assembly of claim 6 wherein each of the openings has an area, the area of the side branch opening (12) being greater than that of each of the remaining openings.

8. The stent assembly of claim 6 wherein the first side branch opening (12) and the second side branch opening are coaxially positioned relative to one another.

9. The stent assembly of claim 3 wherein, the second stent body in the undeployed state comprises a substantially tubular shape, the lumen (6) and the longitudinal axis (3) of the first stent body (1) being common to the second stent body.

10. The stent assembly of claim 9 wherein the first stent body (1) has an end-to-end length and the second stent body has an end-to-end length, the end-to-end length of the second body being less than the end-to-end length of the first stent body (1).

11. The stent assembly of claim 9 wherein the first stent body (1) is comprised of a plurality of interconnected first stent members, adjacent first stent members defining a plurality of first openings through the first stent body in fluid communication with the lumen (6), the second stent body is comprised of a plurality of interconnected second stent members, adjacent second stent members defining a plurality of second openings through the second sent body in fluid communication with the lumen, in the undeployed state at least one of the first stent members being positioned at least partially across at least one second opening.

12. The stent assembly of claim 11 wherein the at least one second opening is the second side branch opening.

13. The stent assembly of claim 12 wherein in the undeploycd state the at least one of the first stent members is not positioned across the second side branch opening.

14. A stent delivery system comprising:
• a catheter, the catheter having a catheter shaft and an expandable balloon; and
• a stent assembly of claim 1
• in the undeploycd state the first stent body being disposed about at least a portion of the balloon, the balloon extending through the lumen, in the undeployed state the second stent body is positioned within the lumen of the first stent body.

15. The system of claim 14 further comprising a retractable sheath, having a retained position and a retracted position, in the retained position at least a portion of the sheath being disposed about at least a portion of the first stent body including the first side branch opening, in the retracted position the sheath being removed from at least the first side branch opening.

16. The system of claim 15 wherein the first stent body is placed in the deployed state by expansion of the balloon, the second stent body being placed in the expanded state by withdrawal of the sheath from the retained position to the retracted position.

## Patentansprüche

1. Stentanordnung mit einem nicht entfalteten Zustand und einem entfalteten Zustand, wobei die Anordnung umfasst:
• einen im Wesentlichen röhrenförmigen ersten Stentkörper (1) der ein Lumen (6) definiert, das innerhalb einer Umfangsebene positioniert ist und eine Längsachse (3) durch diese hindurch aufweist; und
• einen zweiten Stentkörper, wobei in dem nicht entfalteten Zustand der zweite Stentkörper mindestens teilweise innerhalb des Lumens (6) des ersten Stentkörpers (1) positioniert ist, der erste Stentkörper (1) eine erste Seitenastöffnung (12) in Fluidkommunikation mit dem Lumen (6) definiert, der zweite Stentkörper eine Vielzahl von hervorstehenden Seitenastelementen (16) umfasst, die hervorstehenden Elemente (16) eine zweite Seitenastöffnung (12) in Fluidkommunikation mit der ersten Seitenastöffnung und dem Lumen definieren, in dem entfalteten Zustand die hervorstehenden Elemente (16) sich durch die erste Seitenastöffnung (12) erstrecken und einen schiefen Winkel in Bezug auf die Längsachse (3) bilden, wobei der zweite Stentkörper aus der Vielzahl von hervorstehenden Seitenastelementen (16) und einem Montagcring (22) besteht, jedes der hervorstehenden Seitenastelemente (16) ein freies Ende und ein im Eingriff befindliches Ende aufweist, wobei das im Eingriff befindliche Ende mit dem Montagering (22) im Eingriff ist, **dadurch gekennzeichnet, dass** der erste Stentkörper (1) durch mindestens ein an die erste Seitenastöffnung (12) angrenzendes Eingriffsgebiet in dem Montagering (22) in Eingriff ist und der zweite Stentkörper ferner aus einer Vielzahl von Eingriffselementen besteht, wobei die Eingriffselemente sich von dem Montagering (22) zu dem mindestens einen Eingriffsgebiet des ersten Stentkörpers (1) erstrecken.

2. Stentanordnung gemäß Anspruch 1, wobei der erste Stentkörper (I) ein Ballon-expandierbarer Stent ist.

3. Stentanordnung gemäß Anspruch 2, wobei der zweite Stentkörper ein selbstexpandierbarer Stent ist.

4. Stentanordnung gemäß Anspruch 3, wobei der erste Stentkörper (1) mindestens teilweise aus mindestens einem Material aus der Gruppe bestehend aus: rostfreiem Stahl, Platin, Gold, Kobalt, Chrom, Niob, Titan und irgendwelchen Kombinationen oder Legierungen davon aufgebaut ist.

5. Stentanordnung gemäß Anspruch 3, wobei die Vielzahl von hervorstehenden Seitenastelementen (16) mindestens teilweise aus mindestens einem Material aus der Gruppe bestehend aus: Nickel, Titan Niob und irgendwelchen Kombinationen oder Legierungen davon aufgebaut sind.

6. Stentanordnung gemäß Anspruch 1, wobei der erste Stentkörper (1) aus einer Vielzahl von untereinander verbundenen ersten Stentelementen besteht und aneinander angrenzende erste Stentelemente eine Vielzahl von Öffnungen durch den ersten Stentkörper in Fluidkommunikation mit dem Lumen (6) definieren und mindestens eine der Öffnungen die erste Seitenastöffnung (12) ist.

7. Stentanordnung gemäß Anspruch 6, wobei jede der Öffnungen eine Fläche hat und die Fläche der Seitenastöffnung (12) größer als diejenige von jeder der restlichen Öffnungen ist.

8. Stentanordnung gemäß Anspruch 6, wobei die erste Seitenastöffnung (12) und die zweite Seitenastöffnung koaxial in Bezug zueinander positioniert sind.

9. Stentanordnung gemäß Anspruch 3, wobei der zweite Stentkörper in dem nicht entfalteten Zustand eine im Wesentlichen röhrenförmige Form aufweist und das Lumen (6) und die Längsachse (3) des ersten Stentkörpers (1) gemeinsam mit dem zweiten Stentkörper sind.

10. Stentanordnung gemäß Anspruch 9, wobei der erste Stentkörper (1) eine Länge von Ende zu Ende hat und der zweite Stentkörper eine Länge von Ende zu Ende hat und die Länge von Ende zu Ende des zweiten Körpers kleiner ist als die Länge von Ende zu Ende des ersten Stentkörpers (1).

11. Stentanordnung gemäß Anspruch 9, wobei der erste Stentkörper (1) aus einer Vielzahl von untereinander verbundenen ersten Stentelementen besteht, aneinander angrenzende erste Stentelemente eine Vielzahl von Öffnungen durch den ersten Stentkörper in Fluidkommunikation mit dem Lumen (6) definieren, der zweite Stentkörper aus einer Vielzahl von untereinander verbundenen zweiten Stentelementen besteht, aneinander angrenzende zweite Stentelemente eine Vielzahl von Öffnungen durch den zweiten Stentkörper in Fluidkommunikation mit dem Lumen definieren und in dem nicht entfalteten Zustand mindestens eines der ersten Stentelemente mindestens teilweise über mindestens einer zweiten Öffnung positioniert ist.

12. Stentanordnung gemäß Anspruch 11, wobei die mindestens eine zweite Öffnung die zweite Seitenastöffnung ist.

13. Stentanordnung gemäß Anspruch 12, wobei in dem nicht entfalteten Zustand das mindestens eine der ersten Stentelemente nicht über der zweiten Seitenastöffnung positioniert ist.

14. Stentfreisetzungssystem umfassend:
• einen Katheter, wobei der Katheter einen Katheterschaft und einen expandierbaren Ballon aufweist; und
• eine Stentanordnung gemäß Anspruch 1,
• wobei in dem in dem nicht entfalteten Zustand der erste Stentkörper über mindestens einem Teil des Ballons angeordnet ist, der Ballon sich durch das Lumen erstreckt und in dem nicht entfalteten Zustand der zweite Stentkörper innerhalb des Lumens des ersten Stentkörpers positioniert ist.

15. System gemäß Anspruch 14, ferner umfassend eine zurückziehbar Hülle, die eine beibehaltene Position und eine zurückgezogene Position hat, wobei in der beibehaltenen Position mindestens ein Teil der Hülle über mindestens einem die erste Seitenastöffnung beinhaltenden Teil des ersten Stentkörpers angeordnet ist und in der zurückgezogenen Position die Hülle von mindestens der ersten Seitenastöffnung entfernt ist.

16. System gemäß Anspruch 15, wobei der erste Stentkörper durch Expansion in den nicht entfalteten Zustand versetzt wird und der zweite Stentkörper durch Wegziehen der Hülle aus der beibehaltenen Position in die zurückgezogene Position in den expandierten Zustand versetzt wird.

## Revendications

1. Ensemble de stent avec un état non déployé et un état déployé, l'ensemble comprenant:
• un premier corps de stent (1) essentiellement tubulaire qui définit une lumière (6) positionnée dans une plaine circonférentielle et ayant un axe longitudinal (3) à travers cela ; et
• un deuxième corps de stent, dans l'état non déployé le deuxième corps de stent étant positionné au moins partiellement au-dedans de al lumière (6) du premier corps de stent (1), le premier corps de stent (1) définissant une première ouverture de branche latérale (12) en communication fluidique avec la lumière (6), le deuxième corps de stent comportant une pluralité d'éléments de branche latérale (16) en saillie, les éléments en saillie (16) définissant une deuxième ouverture de branche latérale (12) en communication fluidique avec la première ouverture de branche latérale et la lumière, dans l'état déployé les éléments en saillie (16) s'étendant à travers la première ouverture de branche latérale (12)ct formant un angle oblique par rapport à l'axe longitudinal (3), le deuxième corps de stent consistant de la pluralité d'éléments de branche latérale (16) en saillie et un anneau de montage (22), chacun des éléments de branche latérale (16) en saillie ayant une extrémité libre et une extrémité engagée, l'extrémité engagée étant engagée à l'anneau de montage (22), **caractérisé en ce que** le premier corps de stent (1) est engagé dans l'anneau de montage (22) par au moins une région d'engagement adjacente à la première ouverture de branche latérale (12), et que le deuxième corps de stent consiste en outre d'une pluralité d'éléments d'engagement, les éléments d'engagement s'étendant de l'anneau de montage (22) à l'au moins une région d'engagement du premier corps de stent (1).

2. Ensemble de stent selon la revendication 1, dans lequel le premier corps de stent (1) est un stent expansible par ballonnet.

3. Ensemble de stent selon la revendication 2, dans lequel le deuxième corps de stent est un stent auto-expansible.

4. Ensemble de stent selon la revendication 3, dans lequel le premier corps de stent (1) est construit au moins partiellement d'un matériau du groupe consistant de: acier inox, platine, or, cobalt, chrome, niobium, titane et n'importe quels combinaisons ou alliages de cela.

5. Ensemble de stent selon la revendication 3, dans lequel la pluralité d'éléments de branche latérale en saillie (16) sont au moins partiellement construits d'un matériau du groupe consistant de: nickel, titane, niobium et n'importe quels combinaisons ou alliages de cela.

6. Ensemble de stent selon la revendication 1, dans lequel le premier corps de stent (1) consiste d'une pluralité de premiers éléments de stent interconnectés, des premiers éléments de stent adjacents définissant une pluralité d'ouvertures à travers le premier corps de stent en communication fluidique avec la lumière (6), au moins une des ouvertures étant la première ouverture de branche latérale (12).

7. Ensemble de stent selon la revendication 6, dans lequel chacune des ouvertures a une aire et l'aire de la ouverture de branche latérale (12) est plus grande que chacune des ouvertures restantes.

8. Ensemble de stent selon la revendication 6, dans lequel la première ouverture de branche latérale (12) et la deuxième ouverture de branche latérale sont positionnées coaxialement par rapport de l'une à l'autre.

9. Ensemble de stent selon la revendication 3, dans lequel le deuxième corps de stent a une forme essentiellement tubulaire dans l'état non déployé, la lumière (6) et l'axe longitudinal (3) du premier corps de stent (1) étant communs avec le deuxième corps de stent.

10. Ensemble de stent selon la revendication 9, dans lequel le premier corps de stent (1) a une longueur d'une extrémité à l'autre extrémité et le deuxième corps de stent a une longueur d'une extrémité à l'autre extrémité et la longueur d'une extrémité à l'autre extrémité du deuxième corps est plus petite que la longueur d'une extrémité à l'autre extrémité du premier corps de stent (1).

11. Ensemble de stent selon la revendication 9, dans lequel le premier corps de stent (1) consiste d'une pluralité de premiers éléments de stent interconnectés, des premiers éléments de stent adjacents définissant une pluralité de première ouvertures à travers le premier corps de stent en communication fluidique avec la lumière (6), le deuxième corps de stent consiste d'une pluralité de deuxièmes éléments de stent interconnectés, des deuxièmes éléments de stent adjacents définissant une pluralité de deuxièmes ouvertures à travers le deuxième corps de stent en communication fluidique avec la lumière, dans l'état non déployé au moins un des premiers éléments étant positionné au moins partiellement au-dessus d'au moins une deuxième ouverture.

12. Ensemble de stent selon la revendication 11, dans lequel l'au moins une deuxième ouverture est la deuxième ouverture de branche latérale.

13. Ensemble de stent selon la revendication 12, dans lequel dans l'état non déployé l'au moins un des premiers éléments de stent n'est pas positionné au-dessus de la deuxième ouverture de branche latérale.

14. Système de pose de stent, comprenant:
• un cathéter, le cathéter ayant une tige de cathéter et un ballonnet expansible; et
• un ensemble de stent selon la revendication 1,
• dans l'état non déployé le premier corps de stent étant disposé au-dessus d'au moins une partie du ballonnet, le ballonnet s'étendant à travers la lumière, dans l'état non déployé le deuxième corps de stent étant positionné au-dedans de la lumière du premier corps de stent.

15. Système selon la revendication 14, comprenant en outre une gaine retirable qui a une position retenue et une position retirée, dans la position retenue au moins une part de la gaine étant disposée au-dessus d'au moins une partie du premier corps de stent qui inclut la première ouverture de branche latérale, dans la position retirée la gaine étant enlevée d'au moins la première ouverture de branche latérale,

16. Système selon la revendication 15, dans lequel le premier corps de stent est mis dans l'état non déployé par la dilatation du ballonnet, le deuxième corps de stent étant mis dans l'état dilaté par enlèvement de la gaine de la position retenue à la position retirée.
